# EUROPEAN PATENT APPLICATION

(11) **EP 2 055 224 A1**
(43) Date of publication of application: **06.05.2009**
(21) Application number: 07021343.4
(22) Date of filing: 31.10.2007
(51) Int. Cl.: A61B 1/24, G02B 7/36, H04N 5/232

(54) **Autofocus method and system therefor**

(71) Applicant: Orangedental GmbH & Co. KG, 88400 Biberach (DE)
(72) Inventor: Piernitzki, Bernhard, 19089 Kobande (DE)
(74) Representative: Cramphorn, Conrad

(57) **Abstract**

A method of automatically focusing an optical system having an imaging unit with a field of view is disclosed. The method comprises the steps of: illuminating an object of interest by means of a light source of the optical system; obtaining a first image of the field of view of the imaging unit of the optical system containing the object of interest by means of the imaging unit of the optical system; determining the brightness of the first image corresponding to the field of view of the imaging unit of the optical system by means of a control unit of the optical system; deriving the distance of the object of interest from the optical system by means of the control unit on the basis of the brightness determined for the first image; and adjusting the focus position of the optical system to the distance of the object of interest from the optical system. The method has the advantage that even if the optical system is out of focus when obtaining the first image it is possible to determine the total brightness thereof. On the basis of this information it is, furthermore, possible to determine the distance of the object of interest from the optical system and, thus, to provide for a first guess of a range of acceptable focus positions in a fast and easy manner.

## Description

### TECHNICAL FIELD OF THE INVENTION

The invention relates to a method of automatically focussing an optical system and in particular an intraoral camera as well as to such an optical system.

### BACKGROUND OF THE INVENTION

It is becoming more and more common that dentists and oral surgeons use an intraoral camera to obtain images of their patient's teeth, mouths and gums to aid in diagnosis and treatment. The most sophisticated intraoral cameras include an autofocus feature, i.e. the ability to automatically bring the camera into focus. Such an autofocus feature can be provided, for instance, by means of a control device and a miniature motor that focuses the imaging lens of the intraoral camera by moving the imaging lens to the best or optimal focus position, i.e. the position where the sharpest possible image of an object of interest can be obtained. Depending on the distance of the object of interest from the intraoral camera, the imaging lens within the intraoral camera has to be positioned at a certain distance from the detector of the intraoral camera, usually a CCD detector, to provide for a good image.

Conventionally, the focus position of a camera is determined automatically mainly by two methods. According to a first method the distance of the camera (or the objective) to the object to be imaged is measured. The focus distance is chosen to correspond to the distance measured between the camera and the object. It is known to measure the distance to the object to be imaged, for instance, by detecting a reflected ultrasound or infrared light pulse.

A second method is based on the fact that the contrast or sharpness of the object to be imaged generally is at maximum in the focus position of the camera. The contrast of the image of the object of interest is determined by means of an image processing software and the distance between the camera and the object of interest is changed iteratively in the direction of higher contrast until substantially no further improvement of the image quality is discernible.

The second method, i.e. determining the best focus position by means of a contrast optimization, has the disadvantage that in most cases initially one has no information about (i) how far the current focus position is off from the best or optimal focus position and (ii) in which direction the best focus position is with respect to the current focus position. As already mentioned above, conventionally, an iterative approach in the direction of the best focus position is used. However, the relationship between the image quality and the distance of the actual focus position to the best focus position is actually not linear, but rather exponential. In other words, close to the best focus position the image quality changes much more rapidly than further off from the best focus position. As a consequence thereof, it is generally very difficult to find a range of acceptable focus positions around the best focus position as a first "guess", because outside of such a range of acceptable focus positions the image quality changes only gradually. Consequently, it would be advantageous to be able to provide for a first guess of a range of acceptable focus positions in a fast and easy manner.

Thus, the object of the present invention is to provide a method of automatically focusing an optical system, in particular an intraoral camera, as well as such a system providing for the possibility to determine a range of acceptable focus positions around the best focus position in a fast and easy manner.

### SUMMARY OF THE INVENTION

The above object is achieved by a method of automatically focusing an optical system having an imaging unit with a field of view. The method comprises the steps of: illuminating an object of interest by means of a light source of the optical system; obtaining a first image of the field of view of the imaging unit of the optical system containing the object of interest by means of the imaging unit of the optical system; determining the brightness of the first image corresponding to the field of view of the imaging unit of the optical system by means of a control unit of the optical system; deriving the approximate distance of the object of interest from the optical system by means of the control unit on the basis of the brightness determined for the first image; and adjusting the focus position of the optical system to the approximate distance of the object of interest from the optical system.

The invention defined in claim 1 has the advantage that even if the optical system is out of focus when obtaining the first image it is possible to determine the total brightness thereof. On the basis of this information it is, furthermore, possible to determine the approximate distance of the object of interest from the optical system and, thus, to provide for a first guess of a range of acceptable focus positions. Although an iterative method of adjusting the focus by means of contrast optimization might still be necessary, one will appreciate that such a method will be much faster in comparison to conventional methods when provided with a first guess of a range of acceptable focus positions by the method according to the present invention.

Preferably, the method comprises the further step of optimizing the focus position by means of a contrast optimization procedure implemented in the control unit of the optical system, after the focus position of the optical system has been adjusted to the approximate distance of the object of interest from the optical system.

According to a preferred embodiment the method further comprises the steps of obtaining a further image with the light source being switched off and subtracting this further image from the first image in order to obtain a brightness-corrected image, wherein the brightness-corrected image is used for determining the approximate distance of the object of interest from the optical system.

Preferably, the approximate distance of the object of interest from the optical system is determined on the basis of a look-up table correlating the brightness of an object of interest measured by the imaging unit of the optical system with the distance of the object of interest from the optical system.

According to a preferred embodiment the object of interest is illuminated by at least one LED.

Furthermore, the object of the invention is achieved by an optical system. The optical system comprises an imaging unit having a field of view and configured for obtaining a first image of the field of view. The optical system further comprises a light source for illuminating an object of interest in the field of view of the imaging unit of the optical system and a control unit. The control unit is configured for determining the brightness of the first image corresponding to the field of view of the imaging unit of the optical system, deriving the approximate distance of the object of interest from the optical system on the basis of the brightness determined for the first image, and adjusting the focus position of the optical system to the approximate distance of the object of interest from the optical system.

### BRIEF DESCRIPTION OF THE DRAWING

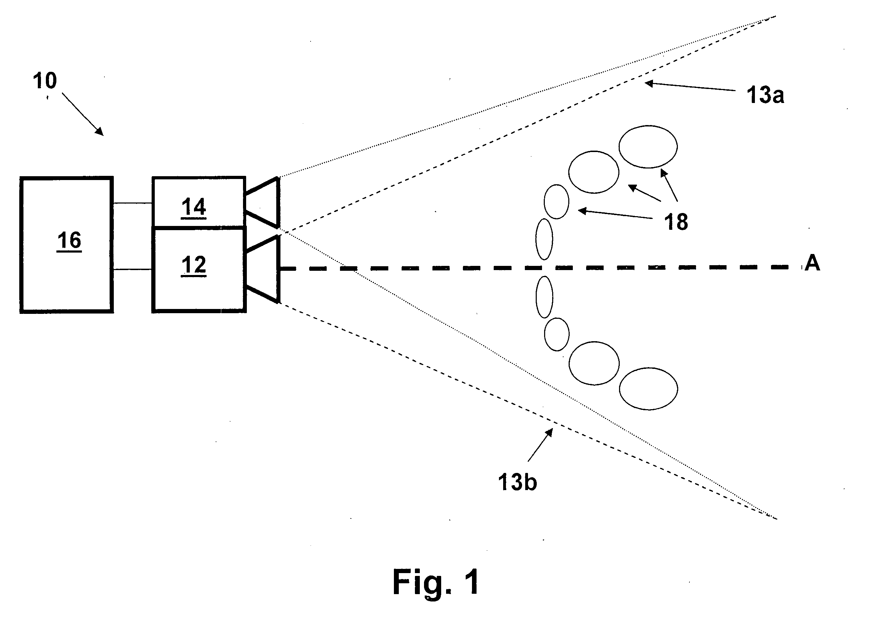

FIGURE 1 shows schematically a preferred embodiment of an optical system for performing the method according to the present invention.

### DETAILED DESCRIPTION OF PREFERRED EMBODIMENTS

A preferred embodiment of the present invention is shown in figure 1. An optical system 10, e.g. a hand-held intraoral camera, comprises a conventional imaging unit 12 consisting, in particular, of an imaging lens, a detector, such as a CCD detector, and means to adjust the distance between the imaging lens and the detector, e.g. a miniature motor. The optical system 10 furthermore comprises a control unit 16 for controlling the components of the imaging unit 12 and processing the images provided by the detector thereof. The control unit 16 of the optical system 10 is furthermore connected with a light source 14.

The imaging unit 12 of the optical system is configured to image a certain field of view around objects of interest 18, e.g. the dental region of a patient. The field of view of the imaging unit 12 is defined by a central symmetry axis A and an angular extension, which, in turn, is defined, for instance, by the lines of sights 13a and 13b shown in the schematic view of figure 1. The light source 14 of the optical system 10 according to the present invention is configured to illuminate the field of view of the imaging unit 12 such that the objects of interest 18 located therein reflect a portion of the light emitted by the light source 14.

The area illuminated by the light source 14 shown in figure 1 substantially coincides with the field of view of the imaging unit 12. However, preferably the area illuminated by the light source 14 coincides completely with the field of view of the imaging unit 12. It is furthermore preferred that the light source 14 comprises one LED or a plurality of LEDs. In order to provide for a substantially complete correspondence between the area illuminated by the light source 14 and the field of view of the imaging unit 12 these LEDs could be arranged in a ring-like configuration centred around the imaging unit 12.

At a certain distance from the optical system 10 and in particular the imaging unit 12 thereof the light emitted by the light source 14 falls onto at least one object of interest 18 and is being reflected thereby. Within an image taken by the imaging unit 12 of the optical system 10 this reflection will contribute a fractional brightness to the brightness of the image obtained by the imaging unit 12. The person skilled in the art will appreciate that the brightness contributed by the reflection of light at the object of interest 18 primarily will depend on the distance of the object of interest 18 from the imaging unit 12. An object of interest 18 closer to the imaging unit 12 will produce a larger brightness than the same object of interest 18 at a greater distance from the imaging unit 12.

Although the brightness contributed by an illuminated object of interest 18 might depend to some degree on additional factors as well, such as the size of the object of interest 18 illuminated by the light source 14 and the optical properties of the surface thereof, these additional factors can easily be taken into account in cases, where all of the objects of interest 18 to be imaged have similar properties. In the context of an intraoral camera, for instance, the objects of interest 18 primarily are the teeth of a patient which more or less have a similar size and similar reflection properties so that in such a case it is possible to determine a one-to-one relationship between the brightness contributed by a tooth and the distance thereof to the imaging unit 12.

According to the present invention the control unit 16 includes means for determining the approximate distance of an object of interest 18 from the imaging unit 12 for a given illumination provided by the light source 14 on the basis of the brightness contributed by the reflection of the object of interest 18.

For instance, the control unit 16 could include a look-up table containing a plurality of entries listing the brightness contributed by a calibration object of interest known to be similar to the objects of interest 18 to be imaged for a plurality of distances. On the basis of such a look-up table the control unit 16 can determine the approximate distance of an object of interest 18 from the optical system 10 on the basis of the brightness and adjust the focus position accordingly.

In order to eliminate the contribution of any external light sources which could adversely affect the automatic determination of the focus position according to the present invention preferably a further image is taken with the light source 14 being switched off. This further image is being subtracted from the image taken with the light source 14 being switched on. By subtracting the two images with and without an illumination by the light source 14, resulting in a brightness-corrected image, it is possible to determine any contributions to the brightness in the image taken with the light source 14 being switched-on which are not attributable to the light source 14 and thus originate from external light sources.

The present invention as described in detail above is not limited to the particular devices, uses and methodology described as these may vary. For instance, although the present invention has been described above in the context of an intraoral camera, it can also be applied advantageously to other optical systems including an autofocus feature, such as endoscopes. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

## Claims

1. A method of automatically focusing an optical system (10) having an imaging unit (12) with a field of view, wherein the method comprises the steps of:
illuminating an object of interest (18) by means of a light source (14) of the optical system (10);
obtaining a first image of the field of view of the imaging unit (12) of the optical system (10) containing the object of interest (18) by means of the imaging unit (12) of the optical system (10);
determining the brightness of the first image corresponding to the field of view of the imaging unit (12) of the optical system (10) by means of a control unit (16) of the optical system (10);
deriving the approximate distance of the object of interest (18) from the optical system (10) by means of the control unit (16) on the basis of the brightness determined for the first image; and
adjusting the focus position of the optical system (10) to the approximate distance of the object of interest (18) from the optical system (10).

2. The method of claim 1, wherein the method comprises the further step of optimizing the focus position by means of a contrast optimization procedure implemented in the control unit (16) of the optical system (10), after the focus position of the optical system (10) has been adjusted to the approximate distance of the object of interest (18) from the optical system (10).

3. The method of claim 1 or claim 2, wherein the method further comprises the steps of obtaining a further image with the light source (14) being switched off and subtracting this further image from the first image in order to obtain a brightness-corrected image, wherein the brightness-corrected image is used for determining the approximate distance of the object of interest (18) from the optical system (10).

4. The method according to anyone of the preceding claims, wherein the approximate distance of the object of interest (18) from the optical system (10) is determined on the basis of a look-up table correlating the brightness of an object of interest (18) measured by the imaging unit (12) of the optical system (10) with the distance of the object of interest (18) from the optical system (10).

5. The method according to anyone of the preceding claims, wherein the object of interest (18) is illuminated by at least one LED.

6. Optical system (10), comprising:
an imaging unit (12) having a field of view and configured for obtaining a first image of the field of view;
a light source (14) for illuminating an object of interest (18) in the field of view of the imaging unit (12) of the optical system (10); and
a control unit (16) for determining the brightness of the first image corresponding to the field of view of the imaging unit (12) of the optical system (10), deriving the approximate distance of the object of interest (18) from the optical system (10) on the basis of the brightness determined for the first image, and adjusting the focus position of the optical system (10) to the approximate distance of the object of interest (18) from the optical system (10).

7. The system of claim 6, wherein the control unit (16) is further configured to optimize the focus position by means of a contrast optimization procedure implemented, after the focus position of the optical system (10) has been adjusted to the approximate distance of the object of interest (18) from the optical system (10).

8. The system of claim 6 or claim 7, wherein the imaging unit (12) is configured to obtain a further image with the light source (14) being switched off, and wherein the control unit (16) is configured to subtract this further image from the first image in order to obtain a brightness-corrected image and to use the brightness-corrected image for determining the approximate distance of the object of interest (18) from the optical system (10).

9. The system according to anyone of claims 6 to 8, wherein control unit (16) is configured to determine the approximate distance of the object of interest (18) from the optical system (10) on the basis of a look-up table correlating the brightness of an object of interest (18) measured by the imaging unit (12) of the optical system (10) with the distance of the object of interest (18) from the optical system (10).

10. The system according to anyone of claims 6 to 9, wherein the light source (14) illuminates the object of interest (18) by at least one LED.
